(19) **Europäisches Patentamt**

**European Patent Office**

**Office européen des brevets**

(11) **EP 0 550 344 B1**

(12) **FASCICULE DE BREVET EUROPEEN**

(45) Date de publication et mention
de la délivrance du brevet:
**01.05.1996 Bulletin 1996/18**

(51) Int. Cl.$^6$: **A61N 1/39**

(21) Numéro de dépôt: **92403580.1**

(22) Date de dépôt: **30.12.1992**

(54) **Défibrillateur implantable**

Implantierbarer Defibrillator

Implantable defibrillator

(84) Etats contractants désignés:
**BE CH DE ES GB IT LI SE**

(30) Priorité: **31.12.1991 FR 9116364**

(43) Date de publication de la demande:
**07.07.1993 Bulletin 1993/27**

(73) Titulaire: **ELA MEDICAL (Société anonyme)**
**F-92541 Montrouge Cédex (FR)**

(72) Inventeurs:
 • **Nitzsche, Rémi**
  **F-78650 Beynes (FR)**

 • **Limousin, Marcel**
  **F-92120 Montrouge (FR)**
 • **Bonnet, Jean-Luc**
  **F-92170 Vanves (FR)**
 • **Henry, Christine**
  **F-75014 Paris (FR)**

(74) Mandataire: **Laget, Jean-Loup**
  **Cabinet Pierre Loyer**
  **77, rue Boissière**
  **F-75116 Paris (FR)**

(56) Documents cités:
  **EP-A- 0 360 412**      **EP-A- 0 401 962**
  **EP-A- 0 436 517**      **US-A- 4 860 749**

## Description

L'invention concerne un défibrillateur implantable.

Un défibrillateur est un appareil qui applique au coeur des chocs électriques en cas de fibrillation ventriculaire ou de tachycardie ventriculaire. Il est généralement admis qu'un rythme cardiaque supérieur à 300 c/mn par exemple, correspond à une fibrillation ventriculaire justifiant le déclenchement du défibrillateur, et qu'un rythme cardiaque inférieur à 150 c/mn par exemple ne correspond pas à une tachycardie ventriculaire mais plutôt à un rythme sinusal ou à une tachycardie sinusale. Entre ces deux valeurs, le ventricule peut présenter soit une tachycardie ventriculaire, soit une tachycardie supra-ventriculaire, soit une bi-tachycardie, c'est-à-dire une superposition d'une tachycardie ventriculaire et d'une tachycardie supra-ventriculaire.

Un but de l'invention est de déterminer parmi les trois types de tachycardies précités, celui qui affecte le ventricule.

Usuellement, on découpe le spectre des fréquences cardiaques en bandes, de façon à définir différentes classes de tachycardies. Le document EP 0 360 412 définit ainsi une tachycardie bien tolérée entre 150 et 175 c/mn, une tachycardie moyennement tolérée entre 175 et 200 c/mn, et une tachycardie faiblement tolérée entre 200 et 275 c/mn. Au delà de 275 c/mn ce document considère que le coeur est en fibrillation, et en dessous de 150 c/mn que le rythme est sinusal. En fonction des critères qui sont la fréquence élevée, l'écart brusque de fréquence, la stabilité de fréquence, et la fréquence élevée persistante, différentes thérapies sont programmées pour chaque classe de tachycardie. Ces thérapies consistent en stimulations non agressives, stimulations agressives, chocs de cardioversion et chocs de défibrillation. Elles sont appliquées seules ou en succession en fonction de la classe de la tachycardie définie selon la fréquence cardiaque observée.

Cette approche du problème de la tachycardie ventriculaire n'est pas entièrement satisfaisante car elle n'identifie pas le type de tachycardie que présente le ventricule ; tachycardie ventriculaire, tachycardie supra-ventriculaire, ou bi-tachycardie.

Un autre but de l'invention est de proposer un défibrillateur implantable, commandé en fonction de la détermination du type de tachycardie qui affecte le ventricule. Le brevet US 4.860.749 qui correspond au meilleur état de la technique, décrit un appareil recueillant les signaux représentatifs de l'activité cardiaque à la fois dans le ventricule et dans l'oreillette. A l'aide d'un algorithme faisant intervenir plusieurs seuils de fréquence et la comparaison à des délais introduits au préalable pour chaque patient, l'appareil définit différents types de tachycardies. Selon ce brevet, l'intervalle auriculo-ventriculaire AV lors d'une tachycardie pathologique est supérieur à l'intervalle AV existant durant une tachycardie avec association auriculo-ventriculaire en 1 : 1. Lors d'un examen électrophysiologique préliminaire, le médecin détermine une valeur de seuil et l'introduit dans l'appareil. L'algorithme teste les intervalles AV par rapport à cette valeur de seuil pour définir le type de tachycardie rencontré.

Par ailleurs, dans le cas où la fréquence ventriculaire est supérieure à la fréquence auriculaire, le brevet US 4.860.749 conclut automatiquement à la présence d'une tachycardie ventriculaire. Or il est reconnu que ce cas peut correspondre à d'autres situations telles que : une tachycardie sinusale avec des extra-systoles ventriculaires, une tachycardie supra-ventriculaire en 1 : 1 avec perte de détection auriculaire, ou une fibrillation auriculaire avec rythme ventriculaire rapide et perte de détection auriculaire.

De plus, le critère choisi dans ce brevet, qui présume la présence d'une tachycardie ventriculaire lorsque l'intervalle AV est supérieur à la valeur de l'intervalle AV en rythme sinusal, peut conduire à une fausse interprétation dans la mesure où il est reconnu que les tachycardies supra-ventriculaires induisent des variations non prévisibles de l'intervalle AV.

Un autre but de l'invention est de s'affranchir de l'examen électrophysiologique particulier à chaque patient, et d'étudier la stabilité de divers intervalles avant de caractériser un type de tachycardie de façon à éliminer les fausses interprétations ne nécessitant pas la délivrance d'un choc de défibrillation.

L'invention a pour objet un défibrillateur implantable, commandé en fonction du rythme cardiaque, dans lequel on définit un rythme cardiaque lent, en dessous duquel il n'y a pas de tachycardie ventriculaire, un rythme cardiaque rapide, au dessus duquel on est en présence d'une fibrillation ventriculaire, et un rythme intermédiaire, compris entre les deux rythmes lent et rapide et pour lequel une tachycardie ventriculaire est suspectée, et dans lequel, en cas de rythme intermédiaire, on déclenche l'application par le défibrillateur de la thérapie programmée, lorsque sont simultanément remplies les conditions suivantes :

- association auriculo-ventriculaire en 1 : 1,

- accélération du rythme ventriculaire à la suite d'une désynchronisation auriculo-ventriculaire.

Selon une caractéristique de l'invention, le critère d'accélération du rythme ventriculaire est apprécié sur un nombre de cycles cardiaques de préférence égal à 8.

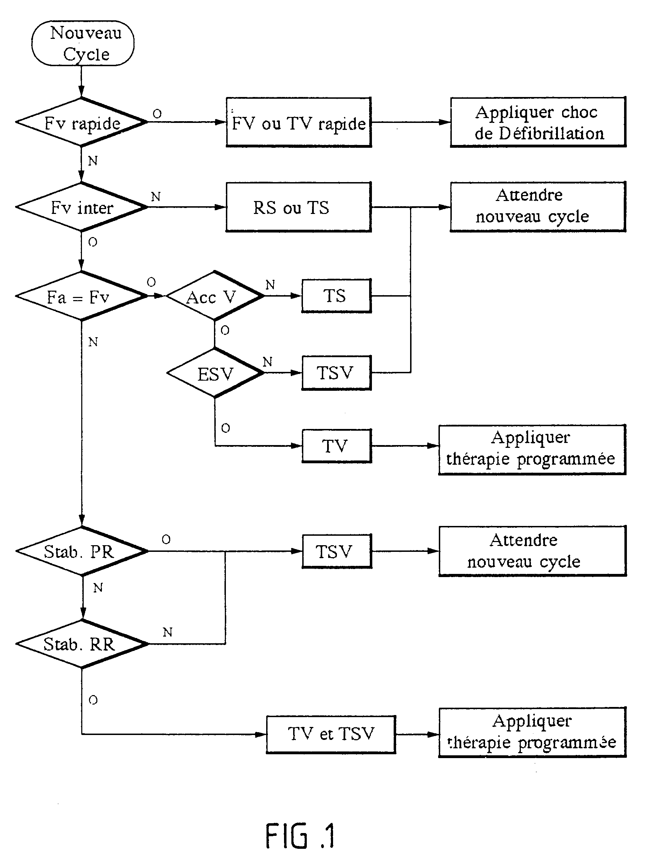

L'invention est décrite ci-après avec référence à la Fig. 1 qui représente l'algorithme de définition des différents types de tachycardies (colonne du milieu) et des actions correspondantes assurées par le défibrillateur (colonne de droite). Sur la Fig. 1, les abréviations ont la signification suivante :

Fv : fréquence ventriculaire,

Fa : fréquence auriculaire,

RS : rythme sinusal,

TS : tachycardie sinusale,

TSV : tachycardie supra-ventriculaire,

TV : tachycardie ventriculaire,

Stab. PR : Stabilité des intervalles PR (entre une onde P et l'onde R suivante) sur un nombre défini de cycles précédents,

Stab. RR : Stabilité des intervalles RR (entre deux ondes R successives) sur un nombre défini de cycles précédents,

FV : Fibrillation ventriculaire

Les mentions ont la signification suivante :

Fv rapide : la fréquence ventriculaire dépasse une fréquence programmée, caractéristique de la fibrillation ventriculaire FV ou des tachycardies ventriculaires rapides (par exemple 300 c/mn),

Fv inter : la fréquence ventriculaire est intermédiaire, c'est-à-dire inférieure à la fréquence programmée caractéristique de la fibrillation ventriculaire, et supérieure à la fréquence de suspicion de la tachycardie ventriculaire (par exemple 150 c/mn)

Fa = Fv : rythmes en association auriculo-ventriculaire en 1 : 1,

Acc. V : le rythme ventriculaire s'est accéléré et est resté accéléré pendant un nombre déterminé de cycles, typiquement 8,

ESV : l'accélération du rythme ventriculaire s'est déclenchée sur une désynchronisation auriculo-ventriculaire qui est définie comme une extra-systole ventriculaire.

Une désynchronisation auriculo-ventriculaire, correspond à l'apparition d'un intervalle PR anormalement long, supérieur à 300 ms par exemple, ou anormalement court, inférieur à 31 ms par exemple.

En suivant l'algorithme de la Fig. 1, en cas de fréquence ventriculaire Fv rapide, le coeur est en fibrillation ventriculaire FV ou en tachycardie ventriculaire rapide TV rapide, et l'action assurée par le défibrillateur est l'application d'un choc de défibrillation.

En cas de fréquence ventriculaire Fv ni rapide, ni intermédiaire, le coeur est en rythme sinusal RS ou en tachycardie sinusale TS, et il convient d'attendre un nouveau cycle.

En cas de fréquence ventriculaire Fv intermédiaire avec rythmes en association auriculo-ventriculaire en 1 : 1 ( Fa = Fv ),

- s'il n'y a pas eu d'accélération du rythme ventriculaire, le coeur est en tachycardie sinusale, et il convient d'attendre un nouveau cycle,

- s'il y a eu accélération du rythme ventriculaire sans désynchronisation auriculo-ventriculaire, le coeur est en tachycardie supra-ventriculaire et il convient d'attendre un nouveau cycle,

- s'il y a eu accélération du rythme ventriculaire avec désynchronisation auriculo-ventriculaire, le coeur est en tachycardie ventriculaire et le défibrillateur applique la thérapie programmée.

En cas de fréquence ventriculaire Fv intermédiaire, et en l'absence d'association auriculo-ventriculaire en 1 :1,

- si les intervalles PR sont stables, le coeur est en tachycardie supra-ventriculaire et il convient d'attendre un nouveau cycle,

- si les intervalles PR ne sont pas stables et si les intervalles RR ne sont pas stables, le coeur est en tachycardie supra-ventriculaire et il convient d'attendre un nouveau cycle,

- si les intervalles PR ne sont pas stables et si les intervalles RR sont stables, le coeur est en tachycardie supra-ventriculaire et. en tachycardie ventriculaire, c'est-à-dire en bi-tachycardie, et le défibrillateur applique la thérapie programmée.

L'analyse faite en application de l'algorithme de la Fig. 1 est effectuée à chaque détection ventriculaire.

En cas de rythme intermédiaire, la thérapie programmée est soit l'application d'une séquence anti-tachycardique ventriculaire automatique, soit l'application d'un chocs de cardioversion, soit l'application d'un choc de défibrillation, soit l'application d'une combinaison de ces actions qui sont graduées d'une faible énergie à une forte énergie.

Il faut remarquer que le procédé de commande du défibrillateur implanté selon l'invention impose une écoute dans la chambre auriculaire et dans la chambre ventriculaire. Il requiert donc deux sondes endocavitaires et les électrodes de défibrillation.

## Revendications

1. Défibrillateur implantable, commandé en fonction du rythme cardiaque, dans lequel on définit un rythme cardiaque lent, en dessous duquel il n'y a pas de tachycardie ventriculaire, un rythme cardiaque rapide, au dessus duquel on est en présence d'une fibrillation ventriculaire, et un rythme intermédiaire, compris entre les deux rythmes lent et rapide et pour lequel une tachycardie ventriculaire est suspectée, et dans lequel, en cas de rythme intermédiaire, on déclenche l'application par le défibrillateur de la thérapie programmée, lorsque sont simultanément remplies les conditions suivantes :

- association auriculo-ventriculaire en 1 : 1,

- accélération du rythme ventriculaire à la suite d'une désynchronisation auriculo-ventriculaire.

2. Défibrillateur selon la revendication 1, caractérisé en ce que le critère d'accélération du rythme ventriculaire est apprécié sur un nombre de cycles cardiaques de préférence égal à 8.

**Claims**

1. An implantable defibrillator, controlled according to the heat rate, in which there are defined a slow heart rate below which there is no ventricular tachycardia, a rapid heart rate above which ventricular fibrillation is present, and an intermediate rate located between the two slow and fast rates and for which ventricular tachycardia is suspected, and in which, in the event of an intermediate rhythm, the application of the programmed therapy by the defibrillator is triggered when the following conditions are met simultaneously:

- atrioventricular association at 1 : 1,

- acceleration of the ventricular rate following atrioventricular desynchronisation.

2. A defibrillator according to Claim 1, characterised in that the criterion of acceleration of the ventricular rate is appreciated over a number of cardiac cycles preferably equal to 8.

**Patentansprüche**

1. Implantierbarer Defibrillator, der abhängig vom Herzrhythmus gesteuert wird und bei dem man einen langsamen Herzrhythmus, unterhalb dessen keine ventrikuläre Tachykardie auftritt, einen schnellen Herzrhythmus, oberhalb dessen ein Herzkammerflimmern auftritt, und einen zwischen dem langsamen und schnellen Rhythmus liegenden mittleren Rhythmus definiert, bei dem das Auftreten einer ventrikulären Tachykardie zweifelhaft ist, wobei im Fall des mittleren Rhythmus die durch den Defibrillator erfolgende Anwendung einer programmierten Therapie ausgelöst wird, wenn die folgenden Bedingungen gleichzeitig erfüllt sind:

- aurikulär-ventrikuläre Assoziation im Verhältnis 1:1,
- Beschleunigung des ventrikulären Rhythmus infolge einer aurikulär-ventrikulären Desynchronisierung.

2. Defibrillator nach Anspruch 1,
   **dadurch gekennzeichnet, daß**
   das Kriterium für die Beschleunigung des ventrikulären Rhythmus über eine Anzahl von vorzugsweise 8 Herzzyklen bewertet wird.

FIG .1